# EUROPÄISCHE PATENTSCHRIFT

(11) **EP 2 229 871 B1**
(45) Veröffentlichungstag und Bekanntmachung des Hinweises auf die Patenterteilung: **16.05.2012**
(21) Anmeldenummer: 10156953.1
(22) Anmeldetag: 18.03.2010
(51) Int. Cl.: A61B 1/303

(54) **Medizinisches Instrument, insbesondere Hysteroskop**
Medical instrument, in particular hysteroscope
Instrument médical, notamment hystéroscope

(30) Priorität: 20.03.2009 DE 102009015392
(43) Veröffentlichungstag der Anmeldung: 22.09.2010
(73) Patentinhaber: Karl Storz GmbH & Co. KG, 78532 Tuttlingen (DE)
(72) Erfinder: Campo, Rudi, 3600 Genk (BE); Doll, Frank, 78607 Talheim (DE)
(74) Vertreter: Weller, Wolfgang

(56) Entgegenhaltungen:
- WO-A2-2008/058157
- DE-A1- 3 942 905
- US-A1- 2005 288 551

## Beschreibung

Die Erfindung betrifft ein medizinisches Instrument insbesondere Hysteroskop, mit einem einen Schaft aufweisenden Schaftteil und einer einen Schaft aufweisenden Optik auf, wobei Schaftteil und Optik längs deren Schäfte derart zueinander verschiebbar sind, dass in einer ersten Stellung der Schaft der Optik den Schaft des Schaftteils distalseitig überragt, und dass in einer zweiten Stellung ein distales Ende des Schaftteiles in etwa auf Höhe eines distalen Endes des Schaftes der Optik zum liegen kommt.

Ein derartiges Instrument ist aus der US 2005/0288 551 A1 bekannt.

Der Schaft der Optik ist in einen äußeren Schaft eingeschoben, so dass beide Schäfte koaxial zueinander verlaufen. Der Schaft der Optik kann relativ zum äußeren Schaft axial verschoben werden und dabei die unterschiedlichen Stellungen einnehmen.

Die WO 2008/058 157 A2 beschreibt ein medizinisches Instrument mit einem Schaft, in dem zahlreiche Kanäle vorhanden sind, wobei in einen der Kanäle der Schaft einer Optik ein- und durchschiebbar ist.

Aus der DE 39 42 905 A1 ist ein Cystoskop-Ureteroskop bekannt, bei dem der Endoskopschaft und die Optik voneinander trennbar sind und ein Instrumentenkanal mit einer gelochten Gummikappe am patientenfemen Ende vorgesehen ist, durch den Behandlungsinstrumente unter Sichtkontrolle einführbar sind.

Hysteroskope dienen zur Inspektion der Gebärmutterhöhle oder zum Einbringen von Instrumenten durch ein Endoskop in die Gebärmutter, z.B. zur Teilresektion submuköser Myome, zur laserchirurgischen Trennung von Septen oder zur intrauterinen Blutungsstillung.

Die Anmelderin bietet in ihrem Katalog GYN 5. Ausgabe 2008, Seiten 69/70 dazu Schäfte an, die entweder zur Untersuchung dienen, oder die für Operationen dienen.

Die Untersuchungsschäfte, die einen runden Querschnitt aufweisen, sind als Optik ausgebildet und dienen beispielsweise zu diagnostischen Zwecken, also zur visuellen Inspektion der Gebärmutter. Dazu wird zumindest das distale Ende des Schaftes einige Zentimeter durch den Gebärmutterhals in die Gebärmutter eingeschoben. Dies ist für die Patientin, insbesondere wenn diese Erkrankungen im Bereich der Gebärmutter aufweist, äußerst schmerzhaft.

Um die Untersuchung besser durchführen zu können, weisen solche Optikschäfte meist zusätzlich einen Spülkanal auf, um ein Spülmedium in die Gebärmutter einzuführen. Dieses Spülmittel dient gleichzeitig zur Dilation, also Aufweitung, der Gebärmutter.

Soll ein Eingriff vorgenommen werden, wird der Untersuchungsschaft abgenommen, und es wird ein Operationsschaft eingeschoben, beispielsweise ein Operationsschaft nach BETTOCCHI.

Dazu muss der Untersuchungsschaft von der Gebärmutter abgezogen werden und anschließend der Operationsschaft eingeführt werden, der wiederum eine Optik trägt und der zusätzlich zumindest noch einen Instrumentenkanal aufweist, durch den ein Instrument hindurchgeführt werden kann, mit dem der eigentliche Eingriff in der Gebärmutter durchgeführt wird.

Sollte also beispielsweise während einer Untersuchung einer Gebärmutter mit einem Untersuchungsschaft festgestellt werden, dass gleich ein operativer Eingriff stattzufinden hat, muss dieser Untersuchungsschaft zunächst abgezogen werden, und anschließend muss der wesentlich durchmessergrößere Operationsschaft eingeführt werden.

Dies ist für die Patientin nicht nur unangenehm, sondern äußerst schmerzhaft.

Ferner müssen zwei unterschiedliche Instrumente bereitgehalten und vorgesehen werden.

Es ist daher Aufgabe der Erfindung, hier Abhilfe zu schaffen und eine Vorrichtung, insbesondere ein Hysteroskop, zu schaffen, mit dem sowohl eine Untersuchung als auch ein operativer Eingriff in möglichst atraumatischer Weise durchgeführt werden kann.

Erfindungsgemäß wird dies dadurch gelöst, dass die Querschnitte der beiden Schäfte so aufeinander abgestimmt sind, dass die beiden aneinanderliegenden Schäfte einen gerundeten, grob ovalären Gesamtquerschnitt ergeben, wobei ein zusätzlicher Instrumentenkanal geschaffen ist, der einmal durch ein U-Profil des distalen Schaftabschnittes und andererseits durch einen Seitenbereich des Schafts der Optik umgrenzt ist.

Diese Maßnahmen haben nun zahlreiche Vorteile.

In der ersten Position kann das Instrument, das aus den beiden Bauteilen Schaftteil und Optik zusammengesetzt ist, als ein Untersuchungsschaft mit relativ kleinem Durchmesser bzw. Querschnitt eingesetzt werden, denn distal ragt in der ersten Stellung nur der Schaft der Optik vor. Hier kann beispielsweise zunächst eine Untersuchung an der Gebärmutter durchgeführt werden, wozu lediglich der Schaft der Optik eingeführt werden muss.

Sollte sich nun herausstellen, dass keine weiteren Manipulationen durchgeführt werden müssen, bleiben die Schäfte in dieser Relativstellung zueinander, und das Instrument kann nach Durchführen der Untersuchung wieder von der Gebärmutter abgezogen werden.

Sollte sich nun herausstellen, dass ein operativer Eingriff notwendig ist, wird der Schaft des Schaftteils so weit nach distal verschoben, bis auch dieser in die Gebärmutter eindringt, und zwar so weit, bis dessen distales Ende etwa auf Höhe des distalen Endes der Optik zum Liegen kommt. Durch den Schaft des Schaftteils ist nun ein Instrumentenkanal geschaffen, durch den ein Instrument in die Gebärmutter eingeführt werden kann, um einen Eingriff durchzuführen. Der bereits in der Gebärmutter befindliche Schaft der Optik verbleibt dort, und über diese Optik kann dann die Manipulation innerhalb der Gebärmutter visuell beobachtet werden.

Im praktischen Einsatz wurde festgestellt, dass der zweite Schaft, nämlich der Schaft des Schaftteils, relativ atraumatisch längs des bereits in die Gebärmutter eingeschobenen Schaftes der Optik eingebracht werden kann.

Dabei sind die Querschnitte der beiden Schäfte so aufeinander abgestimmt, dass die beiden aneinanderliegenden Schäfte einen gerundeten, grob ovalären Gesamtquerschnitt ergeben.

Diese Maßnahme hat den Vorteil, dass die beiden nebeneinander liegenden, in die Gebärmutter eingeschobenen Schäfte einen Körper ergeben, der mit möglichst wenig Schmerzen für die Patientin eingeführt und bei Manipulationen in der Gebärmutter durch den Gebärmutterhals hindurch und hin- und herbewegt werden kann.

Durch diesen Schaft wird der Instrumentenkanal geschaffen, um durch die beiden aneinanderliegenden Schäfte die Manipulation im Inneren der Gebärmutter durchführen zu können. Dabei kann dieser Kanal schon vorher existieren, oder erst durch das Nebeneinanderlegen bzw. -schieben der Schäfte gebildet werden.

In einer weiteren Ausgestaltung der Erfindung weist der Schaft der Optik neben den optischen Bauelementen zumindest einen Saug- und/oder Spülkanal auf.

Diese Maßnahme hat den Vorteil, dass dieser Saug- und/oder Spülkanal bereits, wie zuvor erwähnt, bei der Untersuchung eingesetzt werden kann, beispielsweise um die Gebärmutter zu dilatieren. Dieser Saug- und/oder Spülkanal kann auch bei dem eigentlichen chirurgischen Eingriff herangezogen werden, um Spülflüssigkeiten zu- bzw. wieder abzuführen, gegebenenfalls auch, um abgetrennte Gewebeteile abzuführen.

Somit kommt man mit einer möglichst geringen Anzahl an Bauteilen zurande, um die verschiedenen Manipulationen, sei es Untersuchung oder chirurgischer Eingriff, mit visueller Kontrolle durchführen zu können.

In einer weiteren Ausgestaltung der Erfindung ist der Schaft des Schaftteils als distalseitiger, grob U-förmiger, einseitig offener Schaft ausgebildet, dessen offene Seite an das Querschnittsprofil des Schaftes der Optik anschmiegbar ist.

Diese Maßnahme hat den erheblichen Vorteil, dass der Schaft des Schaftteils sich an die Kontur des Schaftes der Optik anschmiegt, so dass nicht nur der zuvor erwähnte abgerundete Körper entsteht, sondern dass auch die Vorschubbewegung des Schaftes des Schaftteils zum Einführen und zum Bereitstellen des Instrumentenkanals möglichst atraumatisch durchgeführt werden kann.

In einer weiteren Ausgestaltung der Erfindung ist an einem der Bauteile, also entweder an der Optik oder am Schaftteil, eine Führung für den Schaft des anderen Bauteils angeordnet.

Diese Maßnahme hat den Vorteil, dass die Führung an dem einen Schaft dazu dient, dass der andere Schaft, falls dies notwendig ist, einwandfrei geführt werden kann.

Diese einwandfreie Führung führt dazu, dass die beiden Schäfte als eng aneinandergeschmiegter Gesamtkörper verbleiben und nicht über längere Distanz gesehen divergierend auseinanderlaufen.

In einer weiteren Ausgestaltung der Erfindung ist eine Verrastung vorgesehen, die die beiden Bauteile Optik und Schaftteil in den beiden Stellungen miteinander verrastet.

Dies hat den erheblichen handhabmäßigen Vorteil, dass der Operateur, wenn er beispielsweise nur eine Untersuchung durchführen will, die beiden Bauteile in der Stellung miteinander verrastet, nämlich in der ersten Stellung, in der der Schaft der Optik den Schaft des Schaftteils distalseitig überragt. Soll ein operativer Eingriff durchgeführt werden, wird der Schaft des Schaftteils so weit nach distal verschoben, bis er die zweite Stellung erreicht, wobei dies die zweite Rastposition darstellt, d.h. die beiden Schäfte bzw. Bauteile verrasten in dieser Stellung erneut miteinander.

Dies spürt der Operateur und weiß, dass er nunmehr die beiden Schäfte in die für den Eingriff richtigen zweiten Stellung richtige Stellung gebracht hat.

Es versteht sich, dass die vorstehend genannten und die nachstehend noch zu erläuternden Merkmale nicht nur in der jeweils angegebenen Kombination, sondern auch in anderen Kombinationen verwendbar sind, ohne den Rahmen der vorliegenden Erfindung zu verlassen, vorausgesetzt das mindestens die im Patentanspruch 1 definierte Merkmals-Kombination vorhanden ist.

Die Erfindung wird nun anhand der nachfolgenden Ausführungsbeispiele im Zusammenhang mit den beiliegenden Zeichnungen näher beschrieben und erläutert. Es zeigen:
- Fig. 1: eine Seitenansicht einer Optik,
- Fig. 2: einen Querschnitt durch den Schaft der Optik im Bereich deren dista- len Endbereichs,
- Fig. 3: eine Seitenansicht eines Schaftteils,
- Fig. 4: einen entsprechenden Querschnitt durch den Schaft des Schaftteils im Bereich dessen distalen Endes,
- Fig. 5: eine Situation, bei der die Optik so in das Schaftteil von proximal nach distal eingeschoben wurde, dass die beiden Bauteile sich in der ersten Stellung befinden, d.h. der Schaft der Optik überragt distalseitig den Schaft des Schaftteils,
- Fig. 6: eine der Fig. 5 entsprechende Darstellung, bei der sich die beiden Bau- teile in ihrer zweiten Stellung befinden, d.h. das Schaftteil wurde so weit nach distal verschoben, dass die distalen Enden der beiden Schäfte in etwa auf gleicher Höhe zum Liegen kommen,
- Fig. 7: einen Schnitt längs der Linie VII-VII in Fig. 6 und
- Fig. 8: einen Schnitt längs der Linie VIII-VIII in Fig. 6.

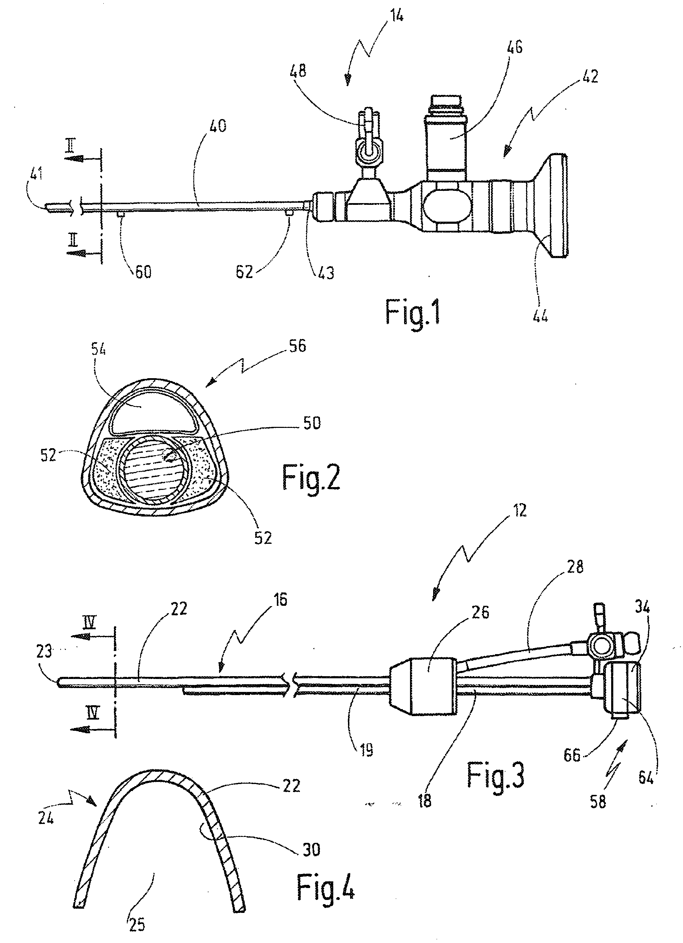

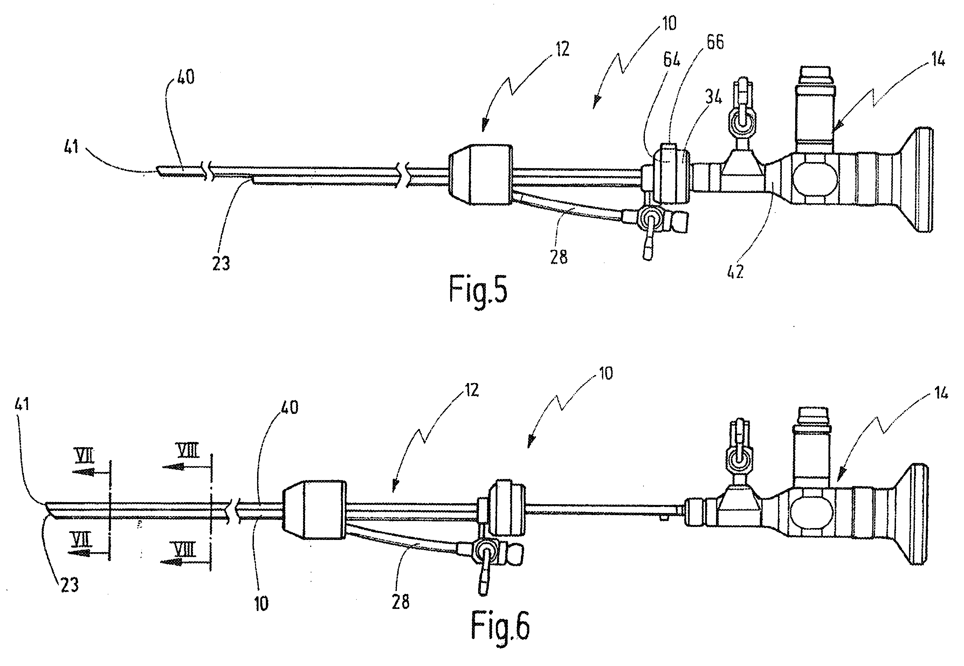

Ein in den Fig. 1 bis 8, insbesondere in den Fig. 5 und 6, dargestelltes erfindungsgemäßes medizinisches Instrument, insbesondere ein Hysteroskop, ist in seiner Gesamtheit mit der Bezugsziffer 10 bezeichnet.

Das Instrument 10, wie es in den Fig. 5 und 6 dargestellt ist, besteht aus einem in Fig. 3 dargestellten Schaftteil 12 und einer in Fig. 1 dargestellten Optik 14.

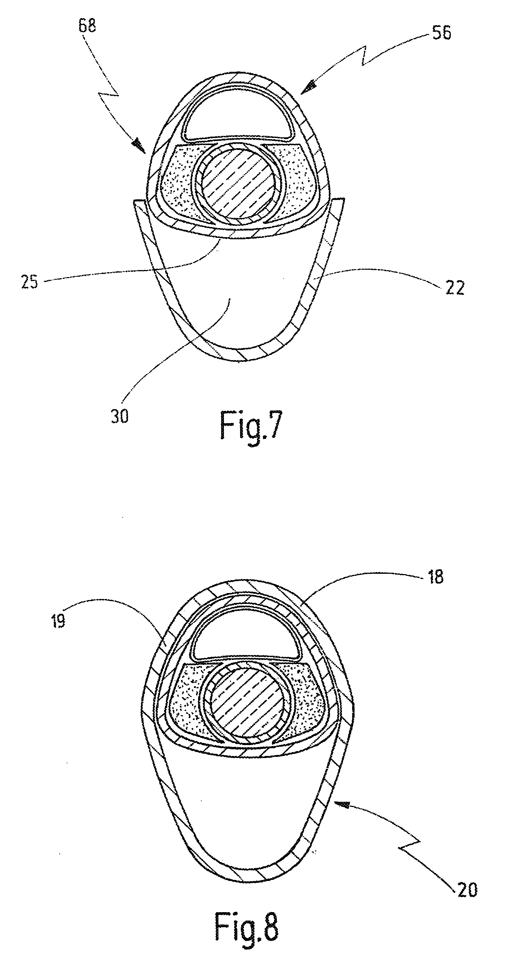

Das in Fig. 3 dargestellte Schaftteil 12 weist einen Schaft 16 auf, der einen proximalen Schaftabschnitt 18 aufweist. Der proximale Schaftabschnitt 18 ist ein geschlossener Schaft und weist, wie aus der Schnittdarstellung von Fig. 8 ersichtlich ist, einen etwa grob ovalären Querschnitt 20 auf.

Distalseitig weist der Schaft 16 einen distalen Schaftabschnitt 22 auf, dessen Querschnitt 24 in Fig. 4 ersichtlich ist. Hier ist zu erkennen, dass der Querschnitt 24 des distalen Schaftabschnittes 22 etwa grob U-förmig ist, und an einer Seite 25 offen ist.

Der distale Schaftabschnitt 22 endet an einem distalen Ende 23.

Der Schaft 16 weist ein Gehäuse 26 auf, von dem seitlich nach proximal ein Anschluss 28 vorsteht, über den ein Instrument durch das Gehäuse 26 in den Schaft 16 eingeführt werden kann.

Der Schaft 16 endet proximalseitig in einem Einführstutzen 34, an dessen Außenseite ein radial beweglicher Druckknopf 66 einer Raste 64 angeordnet ist.

Die in Fig. 1 dargestellte Optik 14 weist einen langerstreckten Schaft 40 auf, der in einem distalen Ende 41 mündet. An seinem proximalen Ende 43 ist der Schaft 40 mit einem Gehäuse 42 verbunden, das proximalseitig ein Okular 44 trägt. Seitlich vom Gehäuse 42 steht zum einen ein Lichtleiteranschluss 46 und zum anderen ein Saug- und Spülanschluss 48 mit einem hier nicht näher bezeichneten Hahn vor.

In dem Schaft 40 ist, wie insbesondere aus der Schnittdarstellung von Fig. 2 ersichtlich ist, ein Optikkanal 50 vorhanden, in dem neben den eigentlichen optischen Elementen, also den Linsen, auch noch Lichtleiter 52 angeordnet sind, wie dies bei einer Optik üblich ist.

Zusätzlich ist in dem Schaft 40 noch ein Saug- und Spülkanal 54 vorhanden, der mit dem Saug-/Spülanschluss 48 in Verbindung steht.

Wie aus der Schnittdarstellung von Fig. 2 ersichtlich ist, weist der Schaft 40 einen Querschnitt 56 auf, der grob dreieckförmig mit sehr sanften bzw. starken Rundungen versehen ist.

Aus der Seitenansicht von Fig. 1 ist noch ersichtlich, dass an der Außenseite des Schaftes 40 voneinander beabstandet zwei Rastnasen 60 und 62 einer Verrastung 58 vorhanden sind.

Die Rastnasen 60 und 62 wirken mit der zuvor beschriebenen Raste 64 bzw. dem Druckknopf 66 am Schaft zusammen, wie dies nachfolgend noch näher erläutert wird.

In Fig. 5 ist eine Situation dargestellt, bei der die Optik 14 von proximal nach distal durch das Schaftteil 12 hindurchgeschoben worden ist. Es sei hier angemerkt, dass die Darstellung des Schaftteils 12 in Fig. 5 und 6 um 180° um die Längsachse gedreht gegenüber der Darstellung von Fig. 3 ist.

Die Optik 14 ist dabei so weit in das Schaftteil 12 eingeschoben, oder im umgekehrten Sinne, das Schaftteil 12 ist so weit auf den Schaft 40 der Optik 14 aufgeschoben, dass der Einführstutzen 34 des Schaftteils 12 in etwa am proximalen Ende des Schaftes 40 der Optik 14 zum Liegen kommt, bzw. eben an das Gehäuse 42 anschlägt.

Dies entspricht der ersten Stellung zwischen Schaftteil 12 und Optik 14.

In dieser ersten Stellung ist die Rastnase 62 der Optik 14 (siehe Fig. 1) mit der Raste 64 am Einführstutzen 34 des Schaftes 16 verrastet.

In dieser ersten Stellung überragt der Schaft 40 der Optik 14 das distale Ende 23 des Schaftes 16 des Schaftteils 12 um einige Zentimeter, wie dies aus Fig. 5 ersichtlich ist.

D.h. das distale Ende 41 des Schaftes 40 ist distal vom distalen Ende 23 des Schaftes 16 beabstandet.

In dieser ersten Stellung kann das Instrument 10 über die Vagina durch den Gebärmutterhals in die Gebärmutter eingeschoben werden, und es kann beispielsweise eine Untersuchung des Innenraums der Gebärmutter durchgeführt werden.

Aufgrund der erfindungsgemäßen Ausgestaltung braucht dazu nur der distalseitig überstehende Abschnitt des Schaftes 40 eingeschoben werden. Nach Anschließen entsprechender Lichtleiter und Anschlüsse an den Saug- und Spülhahn kann der Innenraum der Gebärmutter visuell beobachtet werden, bzw. es kann noch eine Spülflüssigkeit zum Spülen oder Dilatieren der Gebärmutter zugeführt werden.

Soll nun zusätzlich noch ein chirurgischer Eingriff durchgeführt werden, wird das Schaftteil 12 nach distal längs des Schaftes 40 bewegt, wie dies dem Übergang von Fig. 5 zu Fig. 6 entspricht.

Dazu muss der Druckknopf 66 der Raste 44 zunächst gedrückt werden, um die Verrastung in der in Fig. 5 dargestellten Stellung zu lösen. Anschließend kann der Schaft 16 so lange verschoben werden, bis die Raste 64 mit der anderen Rastnase 60 (siehe Fig. 1) an der Außenseite des Schaftes 40 der Optik 14 verrastet.

Diese zweite Stellung entspricht der Darstellung von Fig. 6.

Hier ist ersichtlich, dass das distale Ende 41 des Schaftes 40 der Optik 14 etwa auf gleicher Höhe wie das distale Ende 23 des Schaftes 16 des Schaftteils 12 zum Liegen kommt. Es ist auch ersichtlich, dass die distalen Endflächen der beiden Schäfte 16 und 40 abgerundet sind, so dass sich eine atraumatische Verrundung ergibt, so dass das Gewebe leichter dilatiert bzw. aufdilatiert werden kann. Die resultierende Rundung kann elliptisch, ovalär oder sonst wie ausgebildet sein.

Aus der Schnittdarstellung von Fig. 7 ist ersichtlich, dass sich dabei der seitlich offene distale Schaftabschnitt 22 mit seiner offenen Seite 25 an das äußere Querschnittsprofil des Querschnitts 56 des Schaftes 40 der Optik 14 angelegt bzw. angeschmiegt hat.

Dadurch wird in diesem distalen Endbereich ein zusätzlicher Instrumentenkanal 30 geschaffen, der einmal durch das U-Profil des distalen Schaftabschnittes 22 und andererseits durch einen Seitenbereich des Schaftes 40 der Optik 14 umgrenzt ist.

Durch den nunmehr geschaffenen Instrumentenkanal 30 kann über den seitlichen Anschluss 28 ein Instrument zu- und durchgeführt werden und über das distale Ende 23 hinausgeführt werden, um einen chirurgischen Eingriff in der Gebärmutter durchzuführen. Nach Durchführen des Eingriffs kann das Instrument wieder abgezogen werden.

Je nachdem, wie es günstiger ist, oder wenn noch nachträgliche Untersuchungen durchgeführt werden sollen, kann das Instrument 10 dann in dem in Fig. 6 dargestellten Zusammenbauzustand von der Gebärmutter abgezogen werden, oder es kann zunächst der Schaft aus der in Fig. 6 dargestellten zweiten Stellung in die in Fig. 5 dargestellte erste Stellung verbracht werden.

Es wurde bislang dargestellt, dass der distale Schaftabschnitt 22 als U-förmig einseitig offener Kanal ausgebildet ist, der distale Schaftabschnitt 22 kann aber auch als geschlossener Kanal ausgebildet sein, wobei lediglich wichtig ist, dass dessen Außenprofil, das sich an die Außenseite des Schaftes 40 anschmiegt, dieser Kontur angepasst ist, so dass insgesamt ein Gesamtquerschnitt 68 resultiert, der zu dem abgerundeten Körper führt, durch den sowohl die visuelle Beobachtung als auch der chirurgische Eingriff durchgeführt werden kann.

## Patentansprüche

1. Medizinisches Instrument, insbesondere Hysteroskop, mit einem einen Schaft (16) aufweisenden Schaftteil (12) und einer einen Schaft (40) aufweisenden Optik (14), wobei Schaftteil (12) und Optik (14) längs deren Schäfte (16, 40) derart zueinander verschiebbar sind, dass in einer ersten Stellung der Schaft (40) der Optik (14) den Schaft (16) des Schaftteils (12) distalseitig überragt, und dass in einer zweiten Stellung ein distales Ende (23) des Schaftes (16) des Schaftteils (12) in etwa auf Höhe eines distalen Endes (41) des Schaftes (40) der Optik (14) zum Liegen kommt, **dadurch gekennzeichnet, dass** die Querschnitte (24, 56) der beiden Schäfte (16, 40) so aufeinander abgestimmt sind, dass die beiden aneinanderliegenden Schäfte (16, 40) einen gerundeten, grob ovalären Gesamtquerschnitt (68) ergeben, wobei ein zusätzlicher Instrumentenkanal (30) geschaffen ist, der einmal durch ein U-Profil des distalen Schaftabschnittes (22) und andererseits durch einen Seitenbereich des Schaftes (40) der Optik (14) umgrenzt ist.

2. Medizinisches Instrument nach Anspruch 1 , **dadurch gekennzeichnet, dass** der Schaft (40) der Optik (14) neben den optischen Bauelementen zumindest einen Saug- und/oder Spülkanal (54) aufweist.

3. Medizinisches Instrument nach Anspruch 1 oder 2, **dadurch gekennzeichnet, dass** der Schaft (16) des Schaftteils (12) distalseitig als grob U-förmiger, einseitig offener Schaft (22) ausgebildet ist, dessen offene Seite (25) sich an das Querschnittsprofil des Schaftes (40) der Optik (14) anschmiegt.

4. Medizinisches Instrument nach einem der Ansprüche 1 bis 3, **dadurch gekennzeichnet, dass** an einem der Bauteile, also an Optik (14) oder am Schaftteil (12), eine Führung (19) für den Schaft (40) des anderen Bauteils angeordnet ist.

5. Medizinisches Instrument nach einem der Ansprüche 1 bis 4, **dadurch gekennzeichnet, dass** eine Verrastung (58) vorgesehen ist, die die beiden Bauteile Optik (14) und Schaftteil (12) in den beiden Stellungen miteinander verrastet.

6. Medizinisches Instrument nach Anspruch 5, **dadurch gekennzeichnet, dass** die Verrastung (58) zwei axial voneinander beabstandete Rastnasen (60, 62) an einem der beiden Bauteile und eine Raste (64) an dem anderen Bauteil aufweist.

7. Medizinisches Instrument nach Anspruch 6, **dadurch gekennzeichnet, dass** an der Außenseite des Schaftes (40) der Optik (14) die Rastnasen (60, 62) und an einem Einführstutzen (34) des Schaftteils (12) die Raste (64) angeordnet ist.

8. Medizinisches Instrument nach einem der Ansprüche 1 bis 7, **dadurch gekennzeichnet, dass** die distalen Enden (41, 23) der Schäfte (40, 16) an einer distalen Stirnfläche abgerundet sind.

## Claims

1. Medical instrument, in particular a hysteroscope, with a shaft part (12) having a shaft (16) and an optical system (14) having a shaft (40), in which the shaft part (12) and optical system (14) can be displaced with respect to one another along the shafts (16, 40) thereof such that, in a first position, the shaft (40) of the optical system (14) extends beyond the shaft (16) of the shaft part (12) on the distal side and that, in a second position, a distal end (23) of the shaft (16) of the shaft part (12) comes to rest approximately level with a distal end (41) of the shaft (40) of the optical system (14), **characterized in that** the cross sections (24, 56) of the two shafts (16, 40) are adjusted with respect to each other such that the two adjoining shafts (16, 40) result in a rounded, approximately oval, overall cross section (68), thereby providing an additional instrument channel (30) which is defined by an U-profile of the distal shaft section (22) on the one hand and a lateral area of the shaft (40) of the optical system (14) on the other hand.

2. Medical instrument of Claim 1, **characterized in that** the shaft (40) of the optical system (14) has at least one suction and/or rinsing channel (54) in addition to the optical components.

3. Medical instrument of Claims 1 or 2, **characterized in that** the shaft (16) of the shaft part (12) is, on the distal side, formed as an approximately U-shaped shaft (22) that is open on one side, the open side (25) of which fits closely on the cross-section profile of the shaft (40) of the optical system (14).

4. Medical instrument of anyone of Claims 1 to 3, **characterized in that** on one of the components, that is to say on the optical system (14) or on the shaft part (12), there is arranged a guide (19) for the shaft (40) of the other component.

5. Medical instrument of anyone of Claims 1 to 4, **characterized in that** a latching (58) is provided to latch the two components optical system (14) and shaft part (12) to one another in the two positions.

6. Medical instrument of Claim 5, **characterized in that** the latching (58) has two latching lugs (60, 62), separated axially from one another, on one of the two components and a catch (64) on the other component.

7. Medical instrument of Claim 6, **characterized in that** the latching lugs (60, 62) are arranged on the outer side of the shaft (40) of the optical system (14) and the catch (64) is arranged on a feeding connector (34) of the shaft part (12).

8. Medical instrument of anyone of Claims 1 to 7, **characterized in that** the distal ends (41, 23) of the shafts (40, 16) are rounded on a distal end face.

## Revendications

1. Instrument médical, en particulier hystéroscope, qui comporte une partie de tige (12) présentant une tige (16) et une optique (14) présentant une tige (40), dans lequel la partie de tige (12) et l'optique (14) peuvent être déplacées l'une par rapport à l'autre le long de leur tige (16, 40) de façon telle que dans une première position de la tige (40), l'optique (14) dépasse la tige (16) de la partie de tige (12) côté distal et de façon telle que dans une second position, une extrémité distale (23) de la tige (16) de la partie de tige (12) vient reposer approximativement à la hauteur d'une extrémité distale (41) de la tige (40) de l'optique (14), **caractérisé en ce que** les sections (24, 56) des deux tiges (16, 40) sont adaptées l'une à l'autre de sorte que les deux tiges (16, 40) côte à côte donnent une section totale arrondie, grossièrement ovale (68), un canal d'instrument (30) supplémentaire étant ainsi créé qui est d'une part délimité par un profil en U du segment de tige distal (22) et d'autre part par une zone latérale de la tige (40) de l'optique (14).

2. Instrument médical selon la revendication 1, **caractérisé en ce que** la tige (40) de l'optique (14) présente en plus des éléments optiques de construction au moins un canal d'aspiration et/ou de rinçage (54).

3. Instrument médical selon la revendication 1 ou 2, **caractérisé en ce que** la tige (16) de la partie de tige (12) est formée côté distal en tant que tige (22) ouverte d'un côté, grosso modo en forme de U, dont le côté ouvert (25) s'adapte au profil de section de la tige (40) de l'optique (14).

4. Instrument médical selon l'une des revendications 1 à 3, **caractérisé en ce qu'**au niveau d'un des éléments de construction, donc au niveau de l'optique (14) ou au niveau de la partie de tige (12), est disposé un guide (19) pour la tige (40) de l'autre élément de construction.

5. Instrument médical selon l'une des revendications 1 à 4, **caractérisé en ce qu'**est prévue un enclenchement (58) qui enclenche ensemble les deux éléments de construction optique (14) et partie de tige (12) dans les deux positions.

6. Instrument médical selon la revendication 5, **caractérisé en ce que** l'enclenchement (58) présente deux ergots ou nez d'enclenchement (60, 62) écartés axialement l'un de l'autre au niveau d'un des deux éléments de construction et un ergot ou nez (64) au niveau de l'autre élément de construction.

7. Instrument médical selon la revendication 6, **caractérisé en ce que** les ergots d'enclenchement (60, 62) sont disposés au niveau du côté extérieur de la tige (40) de l'optique (14), et l'ergot (64) est disposé au niveau d'un embout d'introduction (34) de la partie de tige (12).

8. Instrument médical selon l'une des revendications 1 à 7, **caractérisé en ce que** les extrémités distales (41, 23) des tiges (40, 16) sont arrondies au niveau d'une surface frontale distale.
